**Europäisches Patentamt**

**European Patent Office**

**Office européen des brevets**

(19)

(11) Veröffentlichungsnummer : **0 083 775 B2**

(12)

# NEUE EUROPÄISCHE PATENTSCHRIFT

(45) Veröffentlichungstag der neuen Patentschrift :
02.12.92 Patentblatt 92/49

(51) Int. Cl.⁵ : **A61K 9/00,** A61K 47/00,
A61K 31/60

(21) Anmeldenummer : **82111850.2**

(22) Anmeldetag : **21.12.82**

(54) **Gut lösliche 5-Aminosalicylsäure-Arzneimittelzubereitungen.**

(30) Priorität : 23.12.81 DE 3151196

(43) Veröffentlichungstag der Anmeldung :
20.07.83 Patentblatt 83/29

(45) Bekanntmachung des Hinweises auf die
Patenterteilung :
13.09.89 Patentblatt 89/37

(45) Bekanntmachung des Hinweises auf die
Entscheidung über den Einspruch :
02.12.92 Patentblatt 92/49

(84) Benannte Vertragsstaaten :
BE DE FR GB IT

(56) Entgegenhaltungen :
WO-A-81/02671
GB-A- 2 021 409

(56) Entgegenhaltungen :
GB-A- 2 059 768
US-A- 3 131 123
US-A- 4 211 777
Krankenhauspharmazie, 2 Jahrgang nr.
5.1981. S. 123-126
CRC Handbook of chemistry & Physics, 66th
edition, 1985-1986. P. D-146

(73) Patentinhaber : **Bauer, Kurt Heinz, Prof. Dr. rer.
nat.
Hermann-Herder-Strasse 9
W-7800 Freiburg (DE)**

(72) Erfinder : **Bauer, Kurt Heinz, Prof. Dr. rer. nat.
Hermann-Herder-Strasse 9
W-7800 Freiburg (DE)**

(74) Vertreter : **Kraus, Walter, Dr. et al
Patentanwälte Kraus, Weisert & Partner
Thomas-Wimmer-Ring 15
W-8000 München 22 (DE)**

EP 0 083 775 B2

**Beschreibung**

Die Erfindung betritt eine Tabletten-, Filmtabletten-, Dragee-, Kapsel- oder Suppositorien-Zubereitung für die Behandlung von Krankheiten des Colons, welche 5-Aminosalicylsäure enthält.

Die 5-Aminosalicylsäure wurde früher schon zusammen mit der 4-Aminosalicylsäure (PAS) als Tuberculostaticum geprüft, aber für deutlich weniger wirksam befunden. Die 4-Aminosalicylsäure ist auch, verglichen mit der 5-Aminosalicylsäure, wesentlich stabiler und kann deshalb im Gegensatz zu der 5-Aminosalicylsäure ohne Probleme als Natriumsalz appliziert werden.

Zur Behandlung der Colitis ulcerosa und des Morbus Crohn wurde bisher Salazosulfapyridin verwendet. Diese Substanz wird im Darm durch Bakterien oder Enzyme in 5-Aminosalicylsäure und in das Sulfonamid Sulfapyramidin gespalten. Es war lange Zeit unklar, welche der drei Substanzen das wirksame Agens darstellt. Neuere Untersuchungen haben gezeigt, daß die 5-Aminosalicylsäure der wirksame Bestandteil ist, während das Sulfapyramidin hauptsächlich für Nebenwirkungen verantwortlich zeichnet.

Mit der 5-Aminosalicylsäure wurden schon Therapieversuche mit Suppositorien und Einläufen gemacht. Die Anwendung von Suppositorien und insbesondere die von Einläufen ist jedoch unangenehm, insbesondere braucht man für die Verabreichung von Einläufen viel Zeit. Nicht erfolgreich waren bisher die Versuche mit Tabletten. Wegen der schlechten Löslichkeit und/oder der schlechten Stabilität blieb somit die praktische Anwendung der 5-Aminosalicylsäure bisher erfolglos. Aus diesen Gründen wurde die 5-Aminosalicylsäure in der Vergangenheit immer in Form des Salazosulfapyridins verwendet, und auch heute wird in der aktuellen Therapie immer noch Salazosulfapyridin eingesetzt. Man nimmt an, daß die 5-Aminosalicylsäure bei der Spaltung im Dickdarm in "statu nascendi" in feinstverteilter Form anfällt, d.h. in einer Dispersität, die mechanisch durch Mahlen nicht zu bewerkstelligen ist, und auf diese Weise wirksam wird.

Die 5-Aminosalicylsäure (5-Amino-2-hydroxybenzoesäure) ist in den meist gebräuchlichen Lösungsmitteln nur sehr schwer oder nicht löslich. Es ist deshalb praktisch unmöglich, auf diesem Wege die Verbindung zur Gewährleistung einer guten oder zweckentsprechenden Bioverfügbarkeit molekulardispers in Arzneizubereitungen einzuarbeiten. Eine zu diesem Zweck ausreichend gute Löslichkeit besitzt die 5-Aminosalicylsäure nur in starken Alkalien. Bei diesen Bedingungen ist sie jedoch äußerst instabil. Innerhalb von wenigen Minuten verfärbt sich die 5-Aminosalicylsäure bei pH-Werten oberhalb von 10 bis 12 intensiv braun.

In der US-A-4 211 777 und der GB-A-2 021 409 werden gewisse Alkali- und Erdalkalisalze der 5-Aminosalicylsäure beschrieben Arzneimittel, welche 5-Aminosalicylsäure enthalten, werden in diesen Druckschriften nicht erwähnt.

In WO 81/02 671 werden 5-Aminosalicylsäure-Zubereitungen beschrieben. In dieser Druckschrift wird angegeben, daß ein Vorurteil gegen die orale Verabreichung von 5-Aminosalicylsäure besteht und daß ein oral verabreichbares Mittel hergestellt werden kann, wenn man 5-Aminosalicylsäure oder eines ihrer pharmazeutisch annehmbaren Salze oder einen Ester verwendet. Aus dieser Druckschrift geht hervor, daß die Carboxylatsalze nicht untersucht wurden, denn diese sind in wäßriger Lösung unbeständig und können als solche nicht für die orale Verabreichung verwendet werden.

In der GB-A-2 059 768 werden antiinflammatorische Mittel beschrieben, welche Verbindungen mit einem aromatischen Kern und einer oder mehreren hydrophoben Seitenketten enthalten. Die 5-Aminosalicylsäure wird in dieser Druckschrift nicht erwähnt.

Der vorliegenden Erfindung liegt die Aufgabe zugrunde, eine pharmazeutische Zubereitung zur Verfügung zu stellen, die 5-Aminosalicylsäure enthält und bei der sich keine unvertretbaren Stabilitätsprobleme ergeben. Die 5-Aminosalicylsäure soll nach der Applikation in gelöster bzw. feinstverteilter Form am Wirkort aus der Arzneizubereitung freigesetzt werden. Die Anwendung der 5-Aminosalicylsäure in Form von Tabletten, Dragees, Kapseln soll möglich sein.

Gegenstand der Erfindung ist ein Arzneimittel in Form von Tabletten, Filmtabletten, Dragees, Kapseln oder Granulaten für die Behandlung von Krankheiten des Colons, das dadurch gekennzeichnet ist, daß die Zubereitung ein Trockengemisch aus den Einzelstoffen (a) 5-Aminosalicylsäure und (b) einem physiologisch und toxikologisch annehmbaren basischen Hilfsstoff und/oder einem damit vermengten Puffergemisch enthält, wobei der basische Hilfsstoff und/oder das Puffergemisch in 1%iger wäßriger Lösung pH-Werte zwischen 8 und 12 ergeben, mit der Maßgabe, daß die 5-Aminosalicylsäure, der basische Hilfsstoff und/oder das Puffergemisch nicht durch einen Überzug aus Polyvinylpyrrolidon getrennt sind und die Zubereitung magensaftresistent umhüllt ist.

Gegenstand der Erfindung sind weiterhin Suppositorien für die Behandlung von Krankheiten des Colons, die dadurch gekennzeichnet sind, daß die Zubereitung ein Trockengemisch aus den Einzelstoffen (a) 5-Aminosalicylsäure und (b) einem physiologisch und toxikologisch annehmbaren basischen Hilfsstoff und/oder einem damit vermengten Puffergemisch enthält, wobei der basische Hilfsstoff und/oder das Puffergemisch in 1%iger wäßriger Lösung pH-Werte zwischen 8 und 12 ergeben, mit der Maßgabe, daß die 5-Aminosalicylsäure, der basische Hilfsstoff und/oder das Puffergemisch nicht durch einen Überzug aus Polyvinylpyrrolidon getrennt sind.

Gegenstand der Erfindung ist weiterhin ein Arzneimittel in Granulatform, das dadurch gekennzeichnet ist, daß es ein Trockengemisch aus den Einzelstoffen (a) 5-Aminosalicylsäure und (b) einem physiologisch und toxikologisch annehmbaren basischen Hilfsstoff und/oder einem damit vermengten Puffergemisch enthält, wobei die 5-Aminosalicylsäure und der basische Hilfsstoff und/oder das Puffergemisch nicht durch einen Überzug aus Polyvinylpyrrolidon getrennt sind und wobei der basische Hilfsstoff und/oder das Puffergemisch in 1%iger wäßriger Lösung pH-Werte zwischen 8 und 12 ergeben, die Granulatzubereitung eine mittlere Korngröße im Bereich von 0,6 bis 1,5 mm aufweist und das Granulat durch Vermischen der 5-Aminosalicylsäure mit dem basischen Hilfsstoff und/oder dem Puffergemisch und gegebenenfalls festen Arzneimittel-Trägerstoffen und üblichen Zusatzstoffen trocken vermischt wird, das Gemisch zu Briketts verformt wird und die Briketts anschließend zu einem Granulat gebrochen werden.

Gegenstand der Erfindung ist ebenfalls ein Arzneimittel in Granulatform, das dadurch gekennzeichnet ist, daß es ein Trockengemisch aus den Einzelstoffen (a) 5-Aminosalicylsäure und (b) einem physiologisch und toxikologisch annehmbaren basischen Hilfsstoff und/oder einem damit vermengten Puffergemisch enthält, wobei die 5-Aminosalicylsäure und der basische Hilfsstoff und/oder das Puffergemisch nicht durch einen Überzug aus Polyvinylpyrrolidon getrennt sind und wobei der basische Hilfsstoff und/oder das Puffergemisch in 1%iger wäßriger Lösung pH-Werte zwischen 8 und 12 ergeben und wobei die Zubereitung in Granulatform eine durchschnittliche Teilchengröße im Bereich zwischen 0,15 und 0,5 mm aufweist und wobei das Granulat durch Vermischen der 5-Aminosalicylsäure mit dem basischen Hilfsstoff und/ oder dem Puffergemisch und gegebenenfalls festen Arzneimittel-Trägerstoffen und üblichen Zusatzstoffen trocken vermischt wird, das Gemisch zu Briketts verformt wird und die Briketts anschließend zu einem Granulat gebrochen werden.

Die gut löslichen 5-Aminosalicylsäure-Arzneimittelzubereitungen werden hergestellt, indem man die 5-Aminosalicylsäure mit physiologisch und toxikologisch annehmbaren basischen Hilfsstoffen und/oder Puffergemischen, die in 1 %-iger wäßriger Lösung pH-Werte zwischen 8 und 12 ergeben, vermischt und das erhaltene Gemisch in an sich bekannter Weise zu Tabletten, Filmtabletten, Dragees, Kapseln oder Suppositorien verarbeitet.

Bei dem Verfahren verwendet man als basische Hilfsstoffe oder Puffergemische solche, die in 1 %-iger wäßriger Lösung pH-Werte zwischen 8 und 12, vorzugsweise zwischen 9 und 11, aufweisen. Im Prinzip eignen sich alle anorganischen und organischen Verbindungen oder Puffergemische, die ausreichend stabil und ausreichend in Wasser löslich sind, die oben angegebenen pH-Werte in der wäßrigen Lösung ergeben und die physiologisch als Hilfsstoffe akzeptabel, d.h. innerlich ohne nennenswerte Wirkungen oder Nebenwirkungen einnehmbar, sind.

Darunter fallen allgemein Alkali- und einige Erdalkaliverbindungen, insbesondere die Alkali- und Erdalkalisalze, Alkalihydroxide, wie beispielsweise Natrium- oder Kaliumhydroxid, Alkalicarbonate, wie Natrium- oder Kaliumcarbonat, Alkalibicarbonate, wie Natrium- oder Kaliumbicarbonat, Ammoniumhydroxid, Ammoniumcarbonate und Ammoniumbicarbonate sowie Alkaliphosphate und Hydrogenphosphate.

Bevorzugte alkalische Hilfsstoffe sind Natriumcarbonat, sek. Natriumphosphat, sek. Natriumphosphat im Gemisch mit tert.Natriumphosphat, Natronlauge zusammen mit Glycin, Natriumcarbonat, Natriumbicarbonat und Gemische aus Natriumcarbonat und Natriumbicarbonat, Ammoniak, Ammoniumchlorid sowie ein Gemisch aus Ammoniak und Ammoniumchlorid, Calciumhydroxid sowie ein Gemisch aus Calciumhydroxid und Glycin, Natriumglycerinphosphat. Weiterhin können Salze organisch verträglicher Säuren, wie Salze der Zitronensäure oder Weinsäure, beispielsweise Trinatriumcitrat, Dinatriumtartrat, verwendet werden. Anstelle der Natriumsalze können auch die Kaliumsalze verwendet werden. Ein weiterhin bevorzugtes Gemisch ist ein Gemisch aus Natriumcarbonat und Glycin.

Als basische Hilfsstoffe können auch organische Verbindungen, wie Äthanolamin (Colamin), Diäthanolamin, Triäthanolamin, Tri-(hydroxymethyl)-Aminomethan (Tris-Puffer, THAM), N-Methylglukamin, Lysin, Arginin oder Cholin verwendet werden. Von den organischen Verbindungen sind Äthanolamin, Tri-(hydroxymethyl)aminomethan, N-Methylglukamin, Lysin, Arginin und Cholin bevorzugt.

Günstige Voraussetzungen für eine Verwendung sind außerdem die Zulassung als Lebensmittelzusatz, als Arzneimittelhilfsstoff oder zumindest als unbedenklicher Hilfsstoff, ein kleines Molekulargewicht (damit beispielsweise die Tabletten nicht zu groß werden), eine leichte Beschaffbarkeit und ein nicht zu hoher Preis.

Das erfindungsgemäßen Arzneiformen werden in der Weise hergestellt, daß die 5-Aminosalicylsäure mit dem alkalischen Hilfsstoff und/oder Puffergemischen und gegebenenfalls festen Arzneimittel-Trägerstoffen und üblichen Zusatzstoffen trocken vermischt und in an sich bekannter Weise brikettiert. Die Briketts werden anschließend in an sich bekannter Weise zu einem Granulat, beispielsweise zu einem Granulat mit einer mittleren Korngröße im Bereich von 0,6 mm bis 1,5 mm, vorzugsweise von 0,8 mm bis 1,0 mm, gebrochen. Dieses Granulat eignet sich besonders zur Herstellung von Tabletten, Drageekernen oder Kapseln mit Dosierungen von 200 mg bis 900 mg 5-Aminosalicylsäure pro Arzneiformeinheit. Werden feste Arzneiformen mit niedrigeren Dosierungen, beispielsweise mit 30 mg bis 175 mg pro Tablette oder Kapsel, hergestellt, dann sind die Granulate entsprechend feiner zu sieben, und zwar auf mittlere Korngrößen zwischen 0,15 mm bis 0,5 mm. Die Granulate können unter Einsatz üblicher Hilfsstoffe zu Tabletten, Dragees, Kapseln etc. verarbeitet werden. Direkt applizierbare Granulate, die anschließend nicht mehr tablettiert werden sollen, müssen gröber als 1,5 mm gesiebt werden.

Beispiele für feste Trägerstoffe sind mikrokristalline Cellulose, Cellulosepulver, Carboxymethylcellulose, Methylcellulose, Stärke, Milchzucker, Saccharose, Dicalciumphosphat, Calciumsulfat etc.

Beispiele für flüssige, nicht zu einer Salzbildung beitragenden Trägerstoffe sind wasserfreie Öle (Triglyceride), Paraffinöl, Polyäthylenglykole mit den Molekulargewichten 300 bis 600, Glycerin, Propylenglykol etc. Diese flüssigen Trägerstoffe eignen sich u.a. auch als Trägerstoffe zur Abfüllung von 5-Aminosalicylsäure in Weichgelatinekapseln zusammen mit den erfindungsgemäß eingesetzten Zusatzstoffen.

Die Konzentration der basischen Hilfsstoffe bzw. der Puffergemische liegt im allgemeinen im Bereich von 0,5- bis 3-molar, bevorzugt bei 1- bis 1,5-molar, bezogen auf die 5-Aminosalicylsäure. Die Verarbeitung des Gemisches aus 5-Aminosalicylsäure und basischem Hilfsstoff oder Puffergemisch zu der Arzneizubereitung erfolgt ohne Verwendung von Wasser. Die Arzneizubereitung kann erforderlichenfalls, wenn es therapeutisch sinnvoll ist, in an sich bekannter Weise magensaftresistent umhüllt sein. Auf diese Weise wird sichergestellt, daß die Pufferwirksamkeit nicht durch den Einfluß des sauren Magensafts beeinträchtigt wird.

Beim Zusammentreffen mit physiologischen Flüssigkeiten in den Darmbereichen, die entweder in derartige Arzneizubereitungen eindiffundieren oder nach Auflösung der Umhüllung den Inhalt der betreffenden Arzneiform durchsetzen, lösen sich zunächst die Hilfsstoff- oder Puffergemische auf. Durch die hierbei entstehenden pH-Werte, die aus Stabilitätsgründen möglichst weit unter 12 liegen sollten, kann sich die 5-Aminosalicylsäure relativ rasch auflösen.

Die übliche Dosis von Tabletten und Suppositorien liegt heute bei 250 mg und 500 mg (für beide). Die Tagesdosis liegt bei 2 bis 4 g. Es ist möglich, daß sich diese Dosen durch die molekulardisperse Verteilung erniedrigen. Dies kann jedoch erst durch klinische Versuche bewiesen werden.

Die folgenden Beispiele erläutern die Erfindung:

**Beispiel 1**

150,0 g Glycin werden mit 50,0 g Natronlauge 30 %-ig intensiv vermischt und 10 Min. auf einem Wasserbad erwärmt. Anschließend werden aus dieser Mischung 15,0 Wasser herausgetrocknet. Das entstandene trockene Pulver wird unter Lichtschutz zusammen mit 500,0 g 5-Aminosalicylsäure in ca. 1500,0 g bei 50 bis 60°C geschmolzenem Hartfett (Suppositoriengrundmasse) homogen eingerührt. Diese Mischung wird bei 40 bis 50°C in üblicher Weise in Suppositorienformen gegossen, so daß 1000 Zäpfchen mit 2,0 g Bruttogewicht und einem Gehalt von 500 mg 5-Aminosalicylsäure pro Suppositorium entstehen.

**Beispiel 2**

11,0 kg Natriumcarbonat werden mit 1,0 kg Natriumbicarbonat, 25,0 kg 5-Aminosalicylsäure und 4,75 kg Cellulosepulver in bekannter Weise brikettiert und die Briketts anschließend zu einem Granulat mit einer mittleren Korngröße von 0,8 mm gebrochen. Diesem Granulat werden als äußere Phase 0,75 kg Weizenstärke 0,5 kg Magnesiumstearat zugesetzt und mit geeigneten Tablettenpressen zu Tabletten mit einem Bruttogewicht von 0,430 g verpreßt.

Es werden etwa 100.000 Tabletten mit einem 5-Aminosalicylsäuregehalt von 250 mg/Tablette erhalten. Die Tabletten werden in bekannter Weise mit einer Schutzhülle aus Methylcellulose umgeben und zuletzt mit einer magensaftresistenten Filmschicht aus Celluloseacetatphthalat umhüllt.

**Beispiel 3**

4,5 kg Kaliumcarbonat, 1,0 kg Glycin, 6,25 kg 5-Aminosalicylsäure und 2,5 kg sprühgetrocknetes Dicalciumphosphat werden homogen angemischt und mit Hilfe einer Kalanderwalze kompaktiert. Die erhaltenen Schülpen werden durch ein Sieb mit 1 mm lichter Maschenweite geschlagen und als externe Phase 0,5 kg Maisstärke und 0,25 kg Stearinsäurepulver dieser Tablettiermischung zugesetzt. Diese preßfertige Tablettiermischung wird auf geeigneten Tablettiermaschinen zu rund 50 000 dragéegewölbten Kernen mit einem Bruttogewicht von 300 mg verpreßt. Die erhaltenen Drageekerne enthalten pro Stück 125 mg 5-Aminosalicylsäure. Sie werden in üblicher Weise mit einer magensaftresistenen Filmschicht aus Eudragit S[R] ( = anionisches Polymerisat aus Methacrylsäure und Methacrylsäuremethylester) umhüllt und zuletzt mit Zuckersuspension dragiert.

**Beispiel 4**

10,0 kg 5-Aminosalicylsäure und 10,0 kg Ammoniumcarbonat werden in 20,0 kg Pflanzenöl-Wachsmischung suspendiert. Die Suspension wird über einen Dreiwalzenstuhl homogenisiert und anschließend so in Weichgelatinekapseln abgefüllt, daß jede Kapsel 250 mg 5-Aminosalicylsäure enthält. Zuletzt werden die Weichgelatinekapseln noch durch Behandlung mit einer organischen Aldehydlösung magensaftresistent gehärtet.

**Patentansprüche**

1. Tabletten, Filmtabletten, Dragees, Kapseln oder Granulate für die Behandlung von Krankheiten des Colons, dadurch **gekennzeichnet,** daß die Zubereitung ein Trockengemisch aus den Einzelstoffen
   (a) 5-Aminosalicylsäure und
   (b) einem physiologisch und toxikologisch annehmbaren basischen Hilfsstoff und/oder einem damit vermengten Puffergemisch
   enthält, wobei der basische Hilfsstoff und/oder das Puffergemisch in 1%iger wäßriger Lösung pH-Werte zwischen 8 und 12 ergeben, mit der Maßgabe, daß die 5-Aminosalicylsäure, der basische Hilfsstoff und/oder das Puffergemisch nicht durch einen Überzug aus Polyvinylpyrrolidon getrennt sind und die Zubereitung magensaftresistent umhüllt ist.

2. Arzneimittel nach Anspruch 1, dadurch **gekennzeichnet,** daß der basische Hilfsstoff und/oder der Puffer ein Alkalicarbonat, Alkalibicarbonat, Alkalihydroxid, Alkaliphosphat, Ammoniumcarbonat, Erdalkaliphosphat, Ethanolamin, Diethanolamin, Triethanolamin, Tris-(hydroxymethyl)-aminomethan, N-Methylglukamin, Lysin, Arginin, Cholin oder Glycin oder ihre Gemische sind.

3. Arzneimittel nach Anspruch 1, dadurch **gekennzeichnet,** daß der basische Hilfsstoff und/oder der Puffer ein Salz der Zitronensäure oder Weinsäure ist.

4. Arzneimittel nach Anspruch 1, dadurch **gekennzeichnet,** daß der basische Hilfsstoff und/oder der Puffer Natriumcarbonat, sekundäres Natriumphosphat, ein Gemisch aus sekundärem Natriumphosphat und tertiärem Natriumphosphat, ein Gemisch aus Natriumcarbonat und Natriumbicarbonat, ein Gemisch aus Calciumhydroxid und Glycin, Natriumglycerinphosphat, Trinatriumcitrat, Ethanolamin, Tris(hydroxymethyl)-aminomethan, N-Methylglukamin, Lysin, Arginin, Cholin und/oder ihre Gemische sind.

5. Arzneimittel nach Anspruch 1, dadurch **gekennzeichnet,** daß die Menge an basischem Hilfsstoff und/oder Puffer zwischen 0,5 und 3 mol pro mol 5-Aminosalicylsäure beträgt.

6. Arzneimittel nach Anspruch 5, dadurch **gekennzeichnet,** daß die Menge an basischem Hilfsstoff und/oder Puffer zwischen 1 und 1,5 mol pro mol 5-Aminosalicylsäure beträgt.

7. Arzneimittel in Granulatform nach Anspruch 1, dadurch **gekennzeichnet,** daß es ein Gemisch aus den Einzelstoffen
   (a) 5-Aminosalicylsäure und
   (b) einem physiologisch und toxikologisch annehmbaren basischen Hilfsstoff und/oder Puffergemisch
   enthält, wobei der basische Hilfsstoff und/oder das Puffergemisch in 1%iger wäßriger Lösung pH-Werte zwischen 8 und 12 ergeben, die Granulatzubereitung eine mittlere Korngröße im Bereich von 0,6 bis 1,5 mm aufweist und das Granulat durch Vermischen der 5-Aminosalicylsäure mit dem basischen Hilfsstoff und/oder dem Puffergemisch und gegebenenfalls festen Arzneimittel-Trägerstoffen und üblichen Zusatzstoffen trocken vermischt wird, das Gemisch zu Briketts verformt wird und die Briketts anschließend zu einem Granulat gebrochen werden.

8. Arzneimittel in Granulatform nach Anspruch 7, dadurch **gekennzeichnet,** daß es eine mittlere Korngröße im Bereich von 0,8 bis 1,0 mm aufweist.

9. Tabletten, Dragees oder Kapseln nach Anspruch 1, dadurch **gekennzeichnet,** daß sie jeweils 200 mg bis 900 mg 5-Aminosalicylsäure pro Einheitsdosis enthalten, wobei diese Arzneiformen aus den Granulaten nach einem der Ansprüche 7 oder 8 hergestellt worden sind.

10. Arzneimittel in Granulatform nach Anspruch 7, dadurch **gekennzeichnet,** daß es ein Gemisch aus den Einzelstoffen
    (a) 5-Aminosalicylsäure und
    (b) einem physiologisch und toxikologisch annehmbaren basischen Hilfsstoff und/oder Puffergemisch
    enthält, wobei der basische Hilfsstoff und/oder das Puffergemisch in 1%iger wäßriger Lösung pH-Werte zwischen 8 und 12 ergeben und wobei die Zubereitung in Granulatform eine durchschnittliche Teilchengröße im Bereich zwischen 0,15 und 0,5 mm aufweist und wobei das Granulat durch Vermischen der 5-Aminosalicylsäure mit dem basischen Hilfsstoff und/ oder dem Puffergemisch und gegebenenfalls festen Arzneimittel-Trägerstoffen und üblichen Zusatzstoffen trocken vermischt wird, das Gemisch zu Briketts verformt wird und die Briketts anschließend zu einem Granulat gebrochen werden.

11. Tabletten oder Kapseln nach Anspruch 1, dadurch **gekennzeichnet,** daß sie 30 bis 175 mg 5-Aminosalicylsäure pro Einheitsdosis enthalten, wobei diese Zubereitungen aus dem Granulat gemäß Anspruch 10 hergestellt worden sind.

12. Arzneimittel nach Anspruch 1, dadurch **gekenn-**

**zeichnet,** daß der basische Hilfsstoff und/oder der Puffer ein Gemisch aus Natriumcarbonat und Glycin ist.

13. Suppositorien für die Behandlung von Krankheiten des Colons, dadurch **gekennzeichnet,** daß die Zubereitung ein Trockengemisch aus den Einzelstoffen

(a) 5-Aminosalicylsäure und

(b) einem physiologisch und toxikologisch annehmbaren basischen Hilfsstoff und/oder einem damit vermengten Puffergemisch

enthält, wobei der basische Hilfsstoff und/oder das Puffergemisch in 1%iger wäßriger Lösung pH-Werte zwischen 8 und 12 ergeben, mit der Maßgabe, daß die 5-Aminosalicylsäure, der basische Hilfsstoff und/oder das Puffergemisch nicht durch einen Überzug aus Polyvinylpyrrolidon getrennt sind.

**Claims**

1. Tablets, film tablets, coated tablets, capsules or granulates for the treatment of diseases of the colon, characterised in that the preparation contains a dry mixture of the following individual components:

(a) 5-aminosalicylic acid and

(b) a physiologically and toxicologically acceptable basic auxiliary substance and/or a buffer mixture incorporated therewith,

the basic auxiliary substance and/or the buffer mixture giving rise to pH values of from 8 to 12 in a 1% aqueous solution, under the condition that the 5-aminosalicylic acid, the basic auxiliary substance and/or the buffer mixture are not separated by a covering of polyvinyl pyrrolidone and the preparation has a covering which is resistant to gastric juice.

2. Medicament according to Claim 1, characterised in that the basic auxiliary substance and/or the buffer is an alkali metal carbonate, an alkali metal bicarbonate, an alkali metal hydroxide, an alkali metal phosphate, ammonium carbonate, an alkaline earth phosphate, ethanolamine, diethanolamine, triethanolamine, tris-(hydroxymethyl)-aminomethane, N-methylglucamine, lysine, arginine, choline or glycine or mixtures thereof.

3. Medicament according to Claim 1, characterised in that the basic auxiliary substance and/or the buffer is a salt of citric acid or tartaric acid.

4. Medicament according to Claim 1, characterised in that the basic auxiliary substance and/or the buffer is sodium carbonate, secondary sodium phosphate, a mixture of secondary sodium phosphate and tertiary sodium phosphate, a mixture of sodium carbonate and sodium bicarbonate, a mixture of calcium hydroxide and glycine, sodium glycerophosphate, trisodium citrate, ethanolamine, tris-(hydroxymethyl)-aminomethane, N-methylglucamine, lysine, argenine, choline and/or mixtures thereof.

5. Medicament according to Claim 1, characterised in that the quantity of basic auxiliary substance and/or buffer is from 0.5 to 3 mol per mol of 5-aminosalicylic acid.

6. Medicament according to Claim 5, characterised in that the quantity of basic auxiliary substance and/or buffer is from 1 to 1.5 mol per mol of 5-aminosalicylic acid.

7. Medicament in the form of a granulate according to Claim 1, characterised in that it contains a mixture of the following individual components:

(a) 5-aminosalicylic acid and

(b) a physiologically and toxicologically acceptable basic auxiliary substance and/or buffer mixture,

the basic auxiliary substance and/or the buffer mixture giving rise to pH values of from 8 to 12 in a 1% aqueous solution, the preparation of granulate having an average grain size in the range of from 0.6 to 1.5 mm and the granulate being mixed dry by mixing of the 5-aminosalicylic acid with the basic auxiliary substance and/or the buffer mixture and optionally solid medicament carriers and conventional additives, the mixture being formed into briquettes and the briquettes being subsequently broken down into a granulate.

8. Medicament in the form of a granulate according to Claim 7, characterised in that it has an average grain size in the range of from 0.8 to 1.0 mm.

9. Tablets, coated tablets or capsules according to Claim 1, characterised in that they each contain from 200 mg to 900 mg of 5-aminosalicylic acid per unit dose, these forms of medicament having been prepared from the granulates according to one of the Claims 7 or 8.

10. Medicament in the form of a granulate according to Claim 7, characterised in that it contains a mixture of the following individual components:

(a) 5-aminosalicylic acid and

(b) a physiologically and toxicologically acceptable basic auxiliary substance and/or buffer mixture,

the basic auxiliary substance and/or the buffer mixture giving rise to pH values of from 8 to 12 in a 1% aqueous solution and the preparation in the form of a granulate having an average particle size in the range of from 0.15 to 0.5 mm, the granulate being mixed dry by mixing of the 5-aminosalicylic acid with the basic auxiliary substance and/or the buffer mixture and optionally solid medicament carriers and conventional additives, the mixture being formed into briquettes and the briquettes being subsequently broken down into a granulate.

11. Tablets or capsules according to Claim 1, characterised in that they contain from 30 to 175 mg of 5-aminosalicylic acid per unit dose, these preparations having been prepared from the granulate according to Claim 10.

12. Medicament according to Claim 1, characterised in that the basic auxiliary substance and/or the buffer is a mixture of sodium carbonate and glycine.

13. Suppositories for the treatment of diseases of the colon, characterised in that the preparation contains a dry mixture of the following individual components:
    (a) 5-aminosalicylic acid and
    (b) a physiologically and toxicologically acceptable substance and/or a buffer mixture incorporated therewith,
the basic auxiliary substance and/or the buffer mixture giving rise to pH values of from 8 to 12 in 1% aqueous solution, under the condition that the 5-aminosalicylic acid, the basic auxiliary substance and/or the buffer mixture are not separated by a covering of polyvinyl pyrrolidone.

**Revendications**

1. Comprimés, comprimés enrobés, dragées, capsules ou granulés pour le traitement des maladies du côlon, caractérisés en ce que la préparation contient un mélange sec des substances individuelles :
    (a) acide 5-aminosalicylique, et
    (b) un adjuvant basique physiologiquement et toxicologiquement acceptable et/ou un mélange tampon mélangé avec celui-ci,
l'adjuvant basique et/ou le mélange tampon sous forme de solution aqueuse à 1 % produisant des valeurs de pH comprises entre 8 et 12, à la condition que l'acide 5-aminosalicylique, l'adjuvant basique et/ou le mélange tampon ne soient pas séparés par un revêtement de polyvinylpyrrolidone et que la préparation soit enrobée de façon résistante au suc gastrique.

2. Médicament selon la revendication 1, caractérisé en ce que l'adjuvant basique et/ou le tampon est/sont un carbonate alcalin, un bicarbonate alcalin, un hydroxyde alcalin, un phosphate alcalin, le carbonate d'ammonium, un phosphate alcalino-terreux, l'éthanolamine, la diéthanolamine, la triéthanolamine, le tris-(hydroxyméthyl)-amino-méthane, la N-méthylglucamine, la lysine, l'arginine, la choline ou la glycine ou leurs mélanges.

3. Médicament selon la revendication 1, caractérisé en ce que l'adjuvant basique et/ou le tampon est/sont un sel de l'acide citrique ou de l'acide tartrique.

4. Médicament selon la revendication 1, caractérisé en ce que l'adjuvant basique et/ou le tampon est/sont le carbonate de sodium, le phosphate disodique, un mélange de phosphate disodique et de phosphate trisodique, un mélange de carbonate de sodium et de bicarbonate de sodium, un mélange d'hydroxyde de calcium et de glycine, le glycérophosphate de sodium, le citrate trisodique, l'éthanolamine, le tris(hydroxyméthyl)aminométhane, la N-méthylglucamine, la lysine, l'arginine, la choline et/ou leurs mélanges.

5. Médicament selon la revendication 1, caractérisé en ce que la quantité d'adjuvant basique et/ou de tampon est de 0,5 à 3 moles par mole d'acide 5-aminosalicylique.

6. Médicament selon la revendication 5, caractérisé en ce que la quantité d'adjuvant basique et/ou de tampon est de 1 à 1,5 mole par mole d'acide 5-aminosalicylique.

7. Médicament sous forme de granulés selon la revendication 1, caractérisé en ce qu'il contient un mélange des substances individuelles :
    (a) acide 5-aminosalicylique, et
    (b) un adjuvant basique physiologiquement et toxicologiquement acceptable et/ou un mélange tampon,
l'adjuvant basique et/ou le mélange tampon sous forme de solution aqueuse à 1 % produisant des valeurs de pH comprises entre 8 et 12, la préparation de granulés ayant une grosseur moyenne de grains comprise dans la plage de 0,6 à 1,5 mm, les granulés étant obtenus par mélange à sec de l'acide 5-aminosalicylique avec l'adjuvant basique et/ou le mélange tampon et éventuellement des excipients solides pour médicaments et des additifs usuels, le mélange étant façonné en agglomérés et les agglomérés étant fragmentés ensuite en granulés.

8. Médicament sous forme de granulés selon la revendication 7, caractérisé en ce qu'il a une grosseur moyenne de grains comprise dans la plage de 0,8 à 1,0 mm.

9. Comprimés, dragées ou capsules selon la revendication 1, caractérisés en ce qu'ils contiennent chacun 200 mg à 900 mg d'acide 5-aminosalicylique par dose unitaire, ces formes médicamenteuses ayant été préparées à partir des granulés selon la revendication 7 ou 8.

10. Médicament sous forme de granulés selon la revendication 7, caractérisé en ce qu'il contient un mélange des substances individuelles :
    (a) acide 5-aminosalicylique, et
    (b) un adjuvant basique physiologiquement et toxicologiquement acceptable et/ou un mélange tampon,
l'adjuvant basique et/ou le mélange tampon sous forme de solution aqueuse à 1 % produisant des valeurs de pH comprises entre 8 et 12, la préparation sous forme de granulés ayant une grosseur moyenne de grains comprise dans la plage de 0,15 à 0,5 mm, et les granulés étant obtenus par mélange à sec de l'acide 5-aminosalicylique avec l'adjuvant basique et/ou le mélange tampon et éventuellement des excipients solides pour médicaments et des additifs usuels, le mélange étant façonné en agglomérés et les agglomérés étant fragmentés ensuite en granulés.

11. Comprimés ou capsules selon la revendication 1, caractérisés en ce qu'ils contiennent 30 à 175 mg d'acide 5-aminosalicylique par dose unitaire, ces préparations ayant été fabriquées à partir des granulés selon la revendication 10.

12. Médicament selon la revendication 1, caractérisé en ce que l'adjuvant basique et/ou le tampon est/sont un mélange de carbonate de sodium et de glycine.

13. Suppositoires pour le traitement des maladies du côlon, caractérisés en ce que la préparation contient un mélange sec des substances individuelles :
    (a) acide 5-aminosalicylique, et
    (b) un adjuvant basique physiologiquement et toxicologiquement acceptable et/ou un mélange tampon mélangé avec celui-ci,
l'adjuvant basique et/ou le mélange tampon sous forme de solution aqueuse à 1 % produisant des valeurs de pH comprises entre 8 et 12, à la condition que l'acide 5-aminosalicylique, l'adjuvant basique et/ou le mélange tampon ne soient pas séparés par un revêtement de polyvinylpyrrolidone.